# EUROPEAN PATENT APPLICATION

(11) **EP 1 143 246 A1**
(43) Date of publication of application: **10.10.2001**
(21) Application number: 99962510.6
(22) Date of filing: 28.12.1999
(51) Int. Cl.: G01N 33/53, G01N 33/569, C12M 1/34

(54) **SYSTEM FOR IDENTIFYING MICROORGANISM**

(30) Priority: 28.12.1998 JP 37267998
(71) Applicant: KABUSHIKI KAISYA ADVANCE, Chuo-ku Tokyo 103-8354 (JP)
(72) Inventor: SHIBA, Kiyoko, Chiyoda-ku, Tokyo 102-0082 (JP)
(74) Representative: Rinuy, Santarelli
(86) International application number: JP9907427
(87) International publication number: WO0039583

(57) **Abstract**

Disclosed is a simple system for identification of a causative microorganism by determining inflammation markers in the body generated from microbial infection when an infectious disease is caused.

## Description

### TECHNICAL FIELD

The present invention relates to a system for identifying a causative microorganism of an infection by determining various types of inflammation markers present in biological specimens, particularly blood, urine, saliva, nasal mucous, phlegm, exudate from diseased areas, etc. and analyzing the reaction patterns thereof. Upon infection by a microorganism, inflammation markers are produced in the body according to its type and appear in the above biological specimens. By determining the types, amounts of production, molecular structures, etc. of the inflammation markers, it is possible to immediately identify the causative microorganism.

### BACKGROUND ART

In the past, bacteria, mycetes, viruses infection microorganisms such as bacteria, mycetes, viruses have been identified and diagnosed by the method of culturing the blood etc. of the patient and making a determination from the result and the method of detecting an antigen or antibody specific to the causative microorganism from the blood serum etc.

The culturing method includes a method of culturing for several days or more by a medium selecting microorganism and then separating and identifying it and the method of identification from biochemical symptoms based on the metabolism of the microorganisms. Normally, several types of microorganisms are involved in infection, and therefore, in order to confirm the presence of the several types of causative microorganisms suspected, it is necessary to use a number of selective media or biochemical property test kits. Accordingly, a relatively large amount of time is required until the determination. Further, it is sometimes difficult to detect a causative microorganism from the blood. This becomes a major problem in the case of an infectious disease where prompt appropriate determination has to be taken.

As a method of diagnosis of blood serum, there is a method of detecting the microbial antigens, anti-microbial antibodies, microbial components, microbial metabolites, etc., followed by identifying the causative microorganisms. This method of diagnosis of blood serum has the advantage of enabling identification and diagnosis in a shorter time, compared with the blood culturing method, but suffers from numerous problems such as the problem in the specificity to the antigens or components of the microorganism, the fact that microbial metabolites are small in amount, the detection sensitivity is low, and the types identified by test kits are limited, the fact that several days are taken before the appearance of antibodies due to infection in the case of anti-microbial antibodies, and the fact that sometimes antibody-positive responses are given due to existing opportunistic infections.

On the other hand, as the method for determining inflammation markers, there is capillary precipitation, single immunodiffusion, latex turbidity, immunofiltration, immunoturbidity, enzyme immunoassay, etc. These assay methods are being actively used when diagnosing the activity, gravity, and progress of various diseases causing inflammation or tissue disorders. However the above method are not used as the method for identification of the causative microorganisms in infectious diseases.

Infectious diseases are sometimes acute conditions, where life and death hangs in the balance, and therefore, it is necessary to determine the causative microorganisms as fast as possible and apply quick treatment by administration of the appropriate antibiotics. However, as explained above, the culturing method is problematic in the speed of identification of the causative microorganisms at present, while the serum diagnosis method is problematic in the detection sensitivity and specificity and in the range of application etc. Accordingly, the practice is to administer several antibiotics, while guessing the causative microorganisms. Accordingly, various side effects arise and microorganisms tolerant to antibiotics are created.

### DISCLOSURE OF THE INVENTION

The object of the present invention is to solve the above problem by providing a system for immediately and easily identifying all of the causative microorganisms of an infection by determining the state of inflammation markers generated from an infection.

In accordance with the present invention, there is provided a microorganism identification system for identifying a causative microorganism comprising determining inflammation markers in the body generated from microbial infection.

### BRIEF DESCRIPTION OF THE DRAWINGS

The present invention will be explained further below with reference to the drawing:

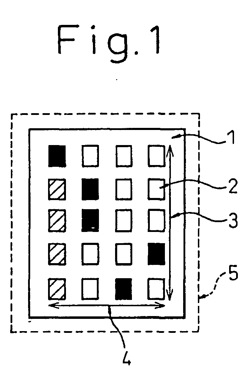

Figure 1 is a view for explaining the present invention.

### BEST MODE FOR CARRYING OUT THE INVENTION

To immediately identify a causative microorganism, the method of examining the white blood cells, which change along with infection, has been devised in addition to the method of detecting the substances relating to infecting microorganisms by applying the antigen-antibody method. However, these methods have problems in terms of convenience since a large amount of biological specimens is required and an equipment not suitable for carrying around is used.

The present inventor found that the types and amounts of production of various inflammation markers appearing in the body and their molecular structures etc. differ depending upon the causative microorganisms of infections. Inflammation markers appear due to inflammation due to major invasive surgery, post-surgical infection, invasion of pathogenic bacteria, etc. However, the pattern of expression of inflammation markers, which is correlated with the fluctuations in the state of the disease, differs depending upon the causes of the inflammation. Further, changes are seen in the molecular structures of the various proteins of the inflammation markers due to the differences in the causative microorganisms.

That is, the present invention determines the types of inflammation markers, the ratio of the amounts produced, the state of change in the molecular structures, etc. and analyzes the pattern of expression to determine whether or not there is infection and, when there is infection, the causative microorganisms is determined.

Inflammation markers are proteins, and therefore, it is possible to prepare antibodies of the inflammation markers. Accordingly, when a plurality of antibodies prepared using, as antigens, the inflammation markers expressed in the body due to infection by various types of microorganisms etc. are provided on a microorganism identification chip and, when biological specimens obtained for a specific purpose are reacted, an antigen-antibody pattern depending upon the causative microorganisms will appear. Note that, when the amount of antigens is very small, the reaction sensitivity can be raised by combining a chemiluminescence system (for example, acridinium is used, as a luminescent substance, or luminol is used, as a chemiluminescent material).

Then, the antigen-antibody reaction pattern of the microorganism identification chip is optically read and analyzed by a simple device programmed with the reaction patterns of various microorganisms in advance, it is possible to accurately identify the causative microorganisms.

Figure 1 will now be explained. Figure 1 shows an example of the configuration of the present invention.

Reference numeral 1 indicates a test chip body made of polyester film, etc.

The overall size is, for example, 25 to 250,000 mm², preferably 100 to 400 mm², with a thickness of 0.5 to 5 mm, but it may be suitably adjusted to another size depending upon the number of amount of the inflammation markers to be determined.

In the Figure, reference numeral 2 indicates one location where an inflammation marker antibody is arranged. The size of one location is, for example, 0.025 to 25 mm² (preferably 1 to 4 mm²). In Figure 1, 20 inflammation marker antibodies are arranged. The distance of arrangement is set to a suitable distance for optically measuring the surface, but a distance of about 0.5 to 2 mm in the left and right and up and down directions may be preferably illustrated.

Each vertical five-location array 3 includes different inflammation marker antibodies, while each horizontal array 4 includes the same inflammation marker changed in the molecular structure, such as molecular weight, isoelectric points, i.e., the array of so-called "isomers".

This square or rectangular uniform array is an example. It is also possible to use a triangular shaped array, diamond shaped array, or other various arrays or even uneven patterns.

The four patterns of isomers and the five types of antibodies are preferable numbers, but more or less of these are also possible depending upon the extent to which the microorganisms to be determined can be confirmed.

Figure 1 shows an example of a pattern of color formation in the case of an actual reaction. The pattern can be read by an optical measurement apparatus (e.g., a CCD camera, infrared or X-ray camera, fluorescent scanner, or PC scanner).

### EXAMPLES

The present invention will now be further explained in detail, but is by no means limited to, the following

### Examples.

### Example 1

The blood and urine of eight cancer patients undergoing major invasive surgery were taken before and directly after surgery and on the second, third, fifth, seventh, 10th, 14th, and 21st days from the day after surgery. Further, each of the patients was administered albumin (ALB) from during surgery to the third day after surgery and was administered a fatty emulsion from the fourth day. The blood and urine thus obtained were separated and purified using cellulose acetate membrane electrophoresis and sodium dodecyl sulfate (SDS) polyacrylamide gel electrophoresis, gel filtration, various types of chromatography, etc. depending upon the objective in order to obtain the α1-acidic glycoprotein, α1-antitrypsin, haptoglobin, CRP, lactoferrin, transferrin, ceruloplasmin, lysozyme, granular elastase, myoperoxidase, IL6, IL8, and other protein-based inflammation markers. The inflammation markers thus obtained were classified into those of the noninfected patients and infected patients. Further, the inflammation markers of the infected patients were classified by causative microorganisms identified by conventional methods.

Next, the inflammation markers were injected, as antigens, into rabbits together with adjuvants. Antihuman inflammation marker rabbit antibodies were prepared by an ordinary method from the blood serum. The various types of antibodies thus obtained were placed in porous tissue in the case of the wet type and were arranged on filter paper in the case of the dry type and then dried to prepare the test chip.

### Example 2

In order to prepare a test solution for reaction with the inflammation marker antibodies placed on the test chip, inflammation marker antibodies were injected, as antigens, into goats to prepare anti-rabbit inflammation marker goat antibodies. After preparing the goat antibodies, labeling substances were bonded for the color forming operation. Luminol or a acridinium ester derivative was used as the label according to an ordinary method to raise the luminescence sensitivity.

This enabled measurement by chemiluminescence and, as a result, a method of raising the sensitivity to 10 to 12 mol was able to be obtained.

### Example 3

The blood and urine of eight cancer patients undergoing major invasive surgery obtained before and directly after surgery and on the second, third, fifth, seventh, 10th, 14th, and 21st days from the day after surgery were dropped on the test chip as biological specimens, then reactions were caused with the test solutions and the luminescence patterns were optically measured.

Further, for confirmation, the total protein was measured by the burette method, the albumin by the BCG method, the C-reactive protein (CRP) by the immunoturbidity, the granular elastase by latex agglutination, and the α1-acidic glycoprotein, α1-antitrypsin, and haptoglobin by a COBASMIRA (Trade mark) automatic system (made by Roche).

The present invention will now be explained using the results of Comparative measurement or identification using the present identification system and a conventional identification system.

### Example 4

The arterial and venous blood and urine of seven cancer patients undergoing major invasive surgery and then not showing complications were obtained before and directly after surgery and on the second, third, fifth, seventh, 10th, 14th, and 21st days from the day after surgery. The degree of invasiveness of the surgery was investigated by the fluctuations in the proteins of the inflammation markers.

Further, all the patients were administered albumin (ALB) from during surgery to the third day after surgery and were administered a fatty emulsion from the fourth day. The arterial and venous blood and urine thus obtained were measured comparatively by the present identification method and the conventional method. As a result, a high correlation was observed in the reaction pattern of the inflammation markers and the usefulness of the present identification method was confirmed.

The results of the measurement by the present identification system are given below. The total protein dropped from immediately after surgery to the second and third day, then returned to the presurgical value from the seventh to 14th day. The albumin fell immediately after surgery, then increased from the first to third days after surgery. The effect of administration of albumin was observed.

The CRP sharply rose immediately after surgery and fluctuated the most wildly. The granular elastase rose rapidly from the first to second day after surgery. The α1-antitrypsin fell directly after surgery, increased from the first day after surgery, and peaked from the fifth to seventh day. The haptoglobin fluctuated in the same way as the α1-antitrypsin, but peaked from the fifth to the 10th day. The β2-microglobulin fell from immediately after surgery to the second day, then increased, peaked from the 10th day to the 14th day, then fell again.

In all inflammation markers, the trends in fluctuation of the arterial blood and venous blood were similar. In diagnosing the condition of the body due to invasive surgery when there are no complications such as infections, the fluctuations in the CRP and granular elastase exhibited the highest correlation with the degree of invasiveness. The other inflammation markers fell immediately after surgery, but it was learned that the peaks of fluctuation became later in the order of α1-antitrypsin, α1-acidic glycoprotein, haptoglobin, and β2-microglobulin.

It could be confirmed from the above results that the present identification method is useful in diagnosing the condition of the body after surgery when there are no complications.

### Example 5

The blood serum and urine of eight cancer patients undergoing major invasive surgery and then showing infectious diseases obtained before and directly after surgery and on the second, third, fifth, seventh, 10th, 14th, and 21st days from the day after surgery were dropped on test chips of the present identification method and reactions of the test solutions were caused, then the chemiluminescence patterns were optically measured. As a result, with post-surgical infection, a high correlation was shown between the degrees of fluctuation of αl-acidic glycoprotein, α1-antitrypsin, and haptoglobin and the state of the infectious disease. For confirmation of this correlation, the inflammation markers in various biological specimens were measured by a conventional method. A Sepharex SP membrane was used for electrophoresis. The amount coated was made 0.8 µl for detection of proteins of the inflammation markers per cm of coating length and 2.4 µl for detection of glycoprotein. The dyeing after electrophoresis was conducted using a Ponso 3R dye for protein dyeing and Schiff's reagent for glycoprotein dyeing. The densitometry was performed by a Densitometer EDC (Helena Institute) at a wavelength of 525 nm for the protein fraction and 570 nm for the glycoprotein fraction.

As a result, since the α1-globulin fraction and the α2-globulin fraction exhibited similar fluctuations as α1-acidic glycoprotein, α1-antitrypsin, and haptoglobin and were correlated with the state of fluctuation of the condition of the infectious disease, it could be confirmed that the present identification method is useful for the diagnosis of the presence of an infectious disease and the degree of recovery.

### Example 6

The blood serum and urine of three cancer patients undergoing major invasive surgery and then showing infectious diseases due to *Pseudomonas aeruginosa* obtained before and directly after surgery and on the second, third, fifth, seventh, 10th, 14th, and 21st days from the day after surgery were dropped on test chips of the present identification method, reactions were caused with the test solutions, then the chemiluminescence patterns were optically measured.

In this Example, test chips provided with antibodies of molecular isomers differing in sugar chains in parallel with the normal inflammation markers were used. As a result, the locations of the inflammation markers differing in sugar chains also strongly reacted in addition to the normal inflammation markers set on the test chips.

In particular, α1-acidic glycoprotein, one of the inflammation markers, exhibited the highest reaction at the locations of the molecular weights of 51 KDa and 46 KDa from immediately after surgery to the third day after surgery. This is believed to be due to the difference caused in the sugar chains of the α1-acidic glycoprotein due to the infection by *Pseudomonas aeruginosa.* Further, the patterns of fluctuation of lactoferrin and transferrin exhibited high correlations with the state of the infectious diseases due to *Pseudomonas aeruginosa.* To confirm the result, the same specimens were fractionated and identified by isoelectric electrophoresis and SDS-polyacrylamide gel electrophoresis. In the case of *Pseudomonas aeruginosa,* known as a causative microorganism for hospital infections, more change appeared in the isoelectric point rather than the molecular size.

That is, a band of a different pH was seen due to the appearance of the α1-acidic glycoprotein different from the normal case. Further, the lactoferrin and transferrin were also detected and disappeared along with the recovery of the infectious diseases, but the patterns of fluctuation exhibited a high correlation similar to the present identification method.

It was able to be confirmed from the above results that the present identification method is useful as an immediate identification method for *Pseudomonas aeruginosa.*

### Example 7

The blood serum and urine of three cancer patients undergoing major invasive surgery and then showing infectious diseases due to MRS (methicillin- resistant Staphylococcus), becoming a problem as a causative microorganism of hospital infections, obtained before and directly after surgery and on the second, third, fifth, seventh, 10th, 14th, and 21st days from the day after surgery were dropped on test chips of the present identification method, the test solutions were made to react, then the chemiluminescence patterns were optically measured.

In this Example, test chips provided with isomers of antibodies of different molecular weights in parallel with the normal inflammation markers were used. As a result, the transferrin and lactoferrin antibody locations arranged on the test chip reacted strongly with not only the normal inflammation markers, but also the same inflammation markers of different molecular weights to give patterns distinctive to MRS infection.

To confirm the results, the same specimens were used for separation and identification of various inflammation markers by their differences in molecular weight by SDS-polyacrylamide gel electrophoresis. In the case of MRS infection, changes were observed (appearance around 82,000 of molecular weight) in the size of the molecules of the proteins transporting the iron of transferrin (molecular weight 80,000) and lactoferrin (molecular weight 80,000) among the inflammation markers. This matched the results of measurement of the present identification method.

It was able to be confirmed from the above results that the present identification method is useful as an immediate identification method for MRS.

### INDUSTRIAL APPLICABILITY

As explained above, the present invention provides a simple system for immediate identification of a causative microorganism by determination and analysis of inflammation markers generated from the cause of onset of infectious diseases.

According to this system, it is possible to administer suitable antibiotics etc. and take other action earlier, and therefore, the side-effects are reduced and the patients can vacate their beds earlier, whereby the medical costs are greatly reduced arising due to long hospitalization, keeping down the appearance of bacteria having tolerance to antibiotics, etc. Therefore the present invention extremely greatly contributes to medicals.

## Claims

1. A microorganism identification system for identifying a causative microorganism comprising determining inflammation markers in a living body generated from microbial infection.

2. A microorganism identification system as claimed in claim 1, wherein the object determined for microorganism identification, including the inflammation marker, is a body fluid, urine, saliva, nasal mucous, phlegm, or exudate from a diseased area.

3. A microorganism identification system as claimed in claim 1, wherein the inflammation markers are proteins of α1-acidic glycoprotein, α1-antitrypsin, haptoglobin, CRP, lactoferrin, transferrin, ceruloplasmin, lysozyme, granular elastase, myoperoxidase, IL6, and IL8.

4. A microorganism identification system as claimed in claim 1, wherein the inflammation markers are combinations of proteins according to claim 3, changed in the structure thereof.

5. A microorganism identification system as claimed in claim 1, wherein the causative microorganism is identified by an antigen-antibody reaction with an antibody according to claim 3 or 4.

6. A microorganism identification system as claimed in claim 1, further comprising chemiluminescence combined to the antigen-antibody reaction according to claim 5, whereby the detection sensitivity is enhanced.

7. A microorganism identification system as claimed in claim 1, wherein a combination of an antibody according to claim 5 and a chemiluminescence system according to claim 6 is placed on a test chip for identification of the causative microorganism, to which the object of determination for identification of the causative microorganism is reacted, whereby the causative microorganism is identified from the reaction pattern thereof.

8. A microorganism identification system as claimed in claim 1, wherein a reaction pattern on a test chip is analyzed by computer processing to thereby identify the causative microorganism.
